# EUROPEAN PATENT APPLICATION

(11) **EP 4 144 849 A1**
(43) Date of publication of application: **08.03.2023**
(21) Application number: 21796151.5
(22) Date of filing: 27.04.2021
(51) Int. Cl.: C12N 15/82, A01H 1/00, A01H 1/02, A01H 5/00

(54) **METHOD FOR OBTAINING WHEAT WITH INCREASED RESISTANCE TO POWDERY MILDEW**

(30) Priority: 27.04.2020 CN 202010343433
(71) Applicant: Suzhou Qi Biodesign biotechnology Company Limited, Jiangsu 215127 (CN)
(72) Inventor: QIU, Jinlong, Beijing 100101 (CN); LI, Shengnan, Beijing 100101 (CN); GAO, Caixia, Beijing 100101 (CN); WANG, Yanpeng, Beijing 100101 (CN)
(74) Representative: Nederlandsch Octrooibureau
(86) International application number: PCT/CN2021/090236
(87) International publication number: WO 2021/218961

(57) **Abstract**

The present invention belongs to the field of plant genetic engineering. In particular, the present invention relates to a method for obtaining wheat with increased resistance to powdery mildew, wherein the wheat has comparable or even increased yield comparable to wild type.

## Description

### Technical Field

The present invention belongs to the field of plant genetic engineering. In particular, the present invention relates to a method for obtaining wheat with increased resistance to powdery mildew, wherein the wheat has comparable or even increased yield comparable to a wild type.

### Background Art

Powdery mildew (Pm) is one of the most important cereal diseases worldwide. Wheat powdery mildew is a worldwide fungal disease caused by the obligate parasitic fungus *Blumeria graminis f. sp tritici.* Powdery mildew generally causes yield loss of 13- 34% in wheat, and up to 50% in severe cases. Breeding resistant wheat varieties is an economic, effective and environmentally friendly strategy to control powdery mildew. The breeding of wheat resistant to powdery mildew is mainly realized by introducing resistance genes of wheat relatives and wild species into the main wheat varieties through hybridization. Since most of these resistance genes belong to race-specific resistance genes, their resistance would be easily broken with the emergence of new physiological races of powdery mildew. At present, the resistance of most wheat varieties (lines) resistant to powdery mildew has been lost or is being lost. Breeding wheat varieties with broad-spectrum and durable resistance is the primary task and challenge in wheat breeding for powdery mildew resistance.

It is an effective way for plants to acquire disease resistance by knocking out Susceptibility genes (S-genes) in the plants to obtain mutants with function deletion of the susceptible genes. MLO gene (Mildew resistance Locus O) in barley is a typical susceptible gene, which has a negative regulatory role against powdery mildew (Acevedo-Garcia et al. 2014). Loss-of-function mutants of the MLO genes exhibit durable and broad-spectrum resistance to almost all races of barley powdery mildew (Piffanelli et al., 2004). At present, the loss-of-function mutants of the MLO genes have been found in several species, such as tomato, potato, pea, pepper and grape, and these mutants also show broad-spectrum and durable resistance to corresponding powdery mildew (Acevedo-Garcia, et al., 2014; Appiano et al., 2015, Feechan, et al., 2008, Zheng, et al., 2013). In conclusion, the function of plant MLO genes to negatively regulate resistance to powdery mildew is highly conserved in evolution. Therefore, based on MLO genes, it is expected to develop a universal strategy to create broad-spectrum and durable powdery mildew resistant plant germplasm.

In hexaploid wheat, there are three copies of the gene with up to 80% homology to barley MLO gene, distributed on 5AL, 4BL and 4DL, named *TaMLO-A1, TaMLO-B1* and *TaMLO-D1,* respectively. The three homologous genes are 98% and 99% similar at the nucleic acid level and amino acid level, respectively. Expression of the wheat *TaMLO-B1* gene in a barley *mlo* mutant can restore the susceptibility of the mutant to barley powdery mildew (Elliott et al., 2002). Furthermore, silencing the *MLO* genes of wheat with VIGS (virus-induced gene silencing) can enhance its powdery mildew resistance (Varallyay et al., 2012). The results show that the function of *MLO* genes in wheat was similar to that of *MLO* gene in barley, and it is conservative in evolution. Due to the multi-copy nature and high similarity of the *MLO* genes in wheat, it is difficult to obtain mutants with simultaneous mutations of three copies by natural mutation and traditional biological means, which may be one of the main reasons why wheat mlo mutants with broad-spectrum resistance to powdery mildew have not been obtained so far. Wang *et al.,* 2014 used TALEN genome editing technology to mutate three copies of MLO gene simultaneously in hexaploid wheat KN199 for the first time, and obtained homozygous mutant of wheat MLO genes, *tamlo-aabbdd,* which showed almost complete resistance to powdery mildew.

However, plant immunity and growth and development are often antagonistic to each other, which is also a long-term challenge in crop breeding for disease resistance. Although *mlo* mutants have been used in powdery mildew resistance breeding and agricultural production of many plants such as barley, tomato and pea, there are still some limitations in the application of *mlo* mutants in plant disease resistance breeding (Acevedo-Garcia, et al., 2014). Mutations in the MLO genes result in a variety of other phenotypes in addition to powdery mildew resistance. Subsequent studies on the KN199 wheat *mlo* triple mutant plants show that the mutant plants exhibit premature leaf senescence at the late growth stage, which results in decrease of yield and will affect its application in breeding and production.

### Summary of the Invention

In one aspect, the invention provides a method of producing a modified wheat plant, the method comprising:
knocking down and/or knocking out *TaMLO-A1, TaMLO-B1* and/or *TaMLO-D1* genes in a wheat plant; and
increasing expression of TaTMT3 protein in the wheat plant,
thereby obtaining a modified wheat plant which has increased resistance to powdery mildew and comparable or preferably increased yield relative to a corresponding wild-type wheat plant.

In some embodiments, in the method, the *TaMLO-A1, TaMLO-B1* and/or *TaMLO-D1* genes in the wheat plant is(are) knocked out or knocked down by introducing antisense RNA, amiRNA, siRNA or shRNA targeting the*TaMLO-A1*, *TaMLO-B1* and/or *TaMLO-D1* genes into the wheat plant.

In some embodiments, in the method, the *TaMLO-A1, TaMLO-B1* and/or *TaMLO-D1* genes in the wheat plant is(are) knocked out or knocked down by introducing a gene editing system, such as a meganuclease, zinc finger nuclease, transcriptional activator-like effector nuclease (TALEN) or CRISPR gene editing system, resulting in mutation(s) in the*TaMLO-A1*, *TaMLO-B1* and/or *TaMLO-D1* genes.

In some embodiments, the method further comprises a step of obtaining a wheat plant homozygous for the mutation(s) in the *TaMLO-A1, TaMLO-B1* and/or *TaMLO-D1* genes.

In some embodiments, in the method, *TaMLO-A1, TaMLO-B1,* and *TaMLO-D1* genes in a wheat plant is(are) knocked out.

In some embodiments, in the method, expression of TaTMT3 protein is increased by introducing an expression construct comprising a nucleotide sequence encoding TaTMT3 protein into the wheat plant, wherein the nucleotide sequence encoding TaTMT3 protein is operably linked to an expression regulatory element.

In one aspect, the invention provides a method for producing a modified wheat plant, the method comprising:
providing a first wheat plant in which *TaMLO-A1, TaMLO-B1,* and/or *TaMLO-D1* gene is(are) knocked down and/or knocked out;
providing a second wheat plant in which the expression of TaTMT3 protein is increased; and
crossing the first wheat plant with the second wheat plant to obtain a modified wheat plant in which *TaMLO-A1, TaMLO-B1* and/or *TaMLO-D1* genes is(are) knocked down and/or knocked out, and the expression of TaTMT3 protein is increased,
wherein the modified wheat plant has increased resistance to powdery mildew and comparable or preferably increased yield relative to a corresponding wild-type wheat plant.

In some embodiments, the first wheat plant has increased resistance to powdery mildew relative to a corresponding wild-type wheat plant.

In some embodiments, the second plant has increased yield relative to a corresponding wild-type wheat plant.

In one aspect, the invention relates to a method for producing a modified wheat plant, the method comprising increasing expression of TaTMT3 protein in a wheat plant, thereby obtaining a modified wheat plant which has an increased yield relative to a corresponding wild-type wheat plant.

In some embodiments, the expression of TaTMT3 protein is increased by introducing an expression construct comprising a nucleotide sequence encoding TaTMT3 protein into the wheat plant, wherein the nucleotide sequence encoding TaTMT3 protein is operably linked to an expression regulatory element.

In one aspect, the invention provides a modified wheat plant or progeny or parts thereof, wherein the wheat plant can be produced or is produced by the method of the invention.

In one aspect, the invention provides a modified wheat plant or progeny or parts thereof, wherein in the modified wheat plant, *TaMLO-A1, TaMLO-B1* and/or *TaMLO-D1* genes is(are) knocked down and/or knocked out, and expression of TaTMT3 protein is increased, and the modified wheat plant has increased resistance to powdery mildew and comparable or preferably increased yield relative to a wild-type wheat plant.

In one aspect, the invention provides a modified wheat plant of the invention or progeny or parts thereof, wherein the modified wheat plant has increased expression of TaTMT3 protein and increased yield relative to a wild-type wheat plant.

In some embodiments of each of the aspects of the invention, the TaMLO-A1 gene encodes the amino acid sequence shown in SEQ ID NO: 2, the TaMLO-B1 gene encodes the amino acid sequence shown in SEQ ID NO: 4, and the TaMLO-D1 gene encodes the amino acid sequence shown in SEQ ID NO: 6.

In some embodiments of each of the aspects of the invention, the TaTMT3 protein is TaTMT3B protein. In some embodiments of each of the aspects of the invention, the TaTMT3B protein comprises the amino acid sequence shown in SEQ ID NO: 8.

In some embodiments of each of the aspects of the invention, the wheat plant is selected from the group consisting of Triticum aestivum, T. aethiopicum, T. araraticum, T. boeoticum, T. carthlicum, T. compactum, T. dicoccoides, T. dicoccum, T. durum, T. ispahanicum, T. karamyschevii, T. macha, T. militinae, T. monococcum, T. polonicum, T. repens, T. spelta, T. sphaerococcum, T. timopheevii, T. turanicum, T. turgidum, T. urartu, T. vavilovii and T. zhukovskyi, and preferably, the wheat plant is Triticum aestivum, in particular cultivar Bobwhite.

### Brief Description of the Drawings

FIG. 1 shows the phenotype of KN199 wheat *mlo* triple mutant plant. A: the *mlo* triple mutant plant had affected growth phenotype compared to the wild type; B: the *mlo* triple mutant plant had decreased yield (1000-grain weight) compared to the wild type.
FIG. 2 shows the phenotype of mutant R33. A: mutant R33 had enhanced resistance to powdery mildew compared to the wild type; B: mutant R33 had no significant difference in growth phenotype from the wild type; C: mutant R33 had significantly increased 1000-grain weight compared to wild type.
FIG. 3 shows the genotype identification of mutant R33. A: the amplified target band in agarose gel electrophoresis; B: mutation pattern of *TaMLO-A1* and *TaMLO-D1* in mutant R33.
FIG. 4 shows the deletion mutation on the B genome of mutant R33. A: a 304 KB fragment was deleted from the B genome of mutant R33; B: two talen target sequences of the deleted fragment: red is the talen recognition site, blue is the mismatch site, and green is the cleavage site Ava II for detection.
FIG. 5 shows the expression analysis of genes near the deleted fragment of R33 mutant. DUG: deletion upstream gene; DDG: deletion downstream gene.
FIG. 6 shows the expression level analysis of TaTMT3 in R33 mutant.
FIG. 7 shows that overexpression of TaTMT3B restored growth defect phenotype of *mlo* mutant. A: TaTMT3B overexpression plant phenotype; B: overexpression of TaTMT3B restored plant height of *mlo* mutant; C: overexpression of TaTMT3B restored yield of *mlo* mutant.

### Detailed Description of the Invention

### 1, Definitions

In the invention, the scientific and technical terms used herein have the meanings commonly understood by those skilled in the art, unless otherwise specified. Also, as used herein, protein and nucleic acid chemistry, molecular biology, cell and tissue culture, microbiology, immunology, and laboratory procedures used herein are terms and routine procedures widely used in the corresponding fields. For example, standard recombinant DNA and molecular cloning techniques used in the present invention are well known to those skilled in the art and are more fully described in: Sambrook, J. Fritsch, E. F. and Maniatis, T., Molecular Cloning: A Laboratory Manual; Cold Spring Harbor Laboratory Press: cold Spring Harbor, 1989 (hereinafter "Sambrook").

As used herein, the term "and/or" encompasses all combinations of items linked by the term, and each combination is to be considered individually listed herein. For example, "A and/or B" encompasses "A", "A and B" and "B". For example, "A, B, and/or C" encompasses "A", "B", "C", "A and B", "A and C", "B and C", and "A and B and C".

As used herein, the term "plant" includes an intact plant and any progeny, cell, tissue, or part of the plant. The term "plant part" includes any part of a plant including, for example but not limited to seeds (including mature seeds, immature embryos without seed coat, and immature seeds); plant cutting; plant cells; plant cell cultures; and plant organs (e.g., pollen, embryos, flowers, fruits, shoots, leaves, roots, stems, and related explants). The plant tissue or plant organ may be seed, callus, or any other population of plant cells organized into structural or functional units. The plant cell or tissue culture is capable of regenerating into plants having the physiological and morphological characteristics of the plant from which the cell or tissue is derived and capable of regenerating into plants having substantially the same genotype as said plant. In contrast, some plant cells are not capable of regenerating into plants. Regenerable cells of plant cells or tissue cultures can be embryos, protoplasts, meristematic cells, callus, pollen, leaves, anthers, roots, root tips, filaments, flowers, kernels, ears, cobs, shells, or stems.

"Progeny" of a plant includes any subsequent generation of the plant.

A "modified plant" includes a plant comprising within its genome an exogenous polynucleotide or comprising a modified gene or expression control sequence. For example, an exogenous polynucleotide can be stably integrated into the genome and inherited in successive generations. The exogenous polynucleotide may be integrated into the genome alone or as part of a recombinant DNA construct. A modified gene or expression control sequence in the plant genome is one which comprises single or multiple nucleotide substitutions, deletions and additions.

A "polynucleotide", "nucleic acid sequence", "nucleotide sequence", or "nucleic acid fragment" is used interchangeably and is a single-or double-stranded RNA or DNA polymer, optionally containing synthetic, non-natural or altered nucleotide bases.

"Polypeptide", "peptide", and "protein" are used interchangeably herein to refer to a polymer of amino acid residues. The term applies to amino acid polymers in which one or more amino acid residues is an artificial chemical analogue of the corresponding naturally occurring amino acid, as well as to naturally occurring amino acid polymers. The terms "polypeptide", "peptide", "amino acid sequence", and "protein" may also include modified forms, including, but not limited to, glycosylation, lipid ligation, sulfation, gamma carboxylation of glutamic acid residues, hydroxylation, and ADP-ribosylation.

Where the term "comprising" is used herein to describe a sequence of a protein or nucleic acid, the protein or nucleic acid may consist of the sequence or may have additional amino acids or nucleotides at one or both ends of the protein or nucleic acid, but still have the activity described herein. Furthermore, it is clear to a person skilled in the art that the methionine at the N-terminus of the polypeptide encoded by the initiation codon may be retained in certain practical situations (e.g., when expressed in a particular expression system) without substantially affecting the function of the polypeptide. Therefore, when describing a particular polypeptide amino acid sequence in the description and claims of the present application, although it may not comprise a methionine encoded by an initiation codon at the N-terminus, a sequence comprising the methionine is also covered herein, and accordingly, the encoding nucleotide sequence thereof may also comprise an initiation codon; vice versa.

Sequence "identity" has its well-recognized meaning in the art and the percentage of sequence identity between two nucleic acid or polypeptide molecules or regions can be calculated using published techniques. Sequence identity can be measured along the entire length of a polynucleotide or polypeptide or along a region of the molecule. While there are many methods of measuring identity between two polynucleotides or polypeptides, the term "identity" is well known to the skilled person in the art (Carrillo, H. &Lipman, D. SIAM J Applied Math 48: 1073 (1988)).

Suitable conservative amino acid substitutions in peptides or proteins are known to those skilled in the art and can generally be made without altering the biological activity of the resulting molecule. In general, those skilled in the art will recognize that single amino acid substitution in the non-essential region of a polypeptide does not substantially alter its biological activity (see, e.g., Watson et al., Molecular Biology of the Gene, 4th Edition, 1987, The Benjamin/Cummings Pub. co., p.224).

As used herein, an "expression construct" refers to a vector, such as a recombinant vector, suitable for expression of a nucleotide sequence of interest in a plant. "Expression" refers to the production of a functional product. For example, expression of a nucleotide sequence can refer to transcription of the nucleotide sequence (e.g., transcription to produce an mRNA or a functional RNA) and/or translation of the RNA into a precursor or mature protein. An "expression construct" of the present invention may be a linear nucleic acid fragment, a circular plasmid, a viral vector, or, in some embodiments, an RNA capable of being translated (e.g., mRNA). An "expression construct" of the present invention may comprise an expression regulatory sequence and a nucleotide sequence of interest from different sources, or an expression regulatory sequence and a nucleotide sequence of interest from the same source but arranged in a manner different from that normally found in nature.

"Expression regulatory sequence" and "expression regulatory element" are used interchangeably and refer to a nucleotide sequence that is located upstream (5 'non-coding sequence), intermediate or downstream (3' non-coding sequence) of a coding sequence and that affects the transcription, RNA processing or stability or translation of the associated coding sequence. Plant expression regulatory elements refer to nucleotide sequences capable of controlling transcription, RNA processing or stability or translation of a nucleotide sequence of interest in a plant.

Expression regulatory sequences may include, but are not limited to, promoters, translation leader sequences, introns, and polyadenylation recognition sequences. "Promoter" refers to a nucleic acid fragment capable of controlling transcription of another nucleic acid fragment. In some embodiments of the invention, the promoter is a promoter capable of controlling transcription of a gene in a plant cell, regardless of whether it is derived from a plant cell. The promoter may be a constitutive promoter or a tissue-specific promoter or a developmentally regulated promoter or an inducible promoter.

As used herein, the term "operably linked" refers to a regulatory element (e.g., but not limited to, a promoter sequence, a transcription termination sequence, etc.) linked to a nucleic acid sequence (e.g., a coding sequence or open reading frame) such that transcription of the nucleotide sequence is controlled and regulated by the transcriptional regulatory element. Techniques for operably linking a regulatory element region to a nucleic acid molecule are known in the art.

### 2. Methods for producing a modified wheat plant

Functional deletion of three copies of wheat MLO gene, *TaMLO-A1, TaMLO-B1* and *TaMLO-D1* genes, such as triple functional knockout mutations, can lead to an enhanced powdery mildew resistance phenotype, but may also lead to a decline in yield. The present inventors have surprisingly found that increasing expression of *TaTMT3B* in the context of functional deletion of MLO genes can obtain a comparable or even increased yield relative to the wild type while maintaining powdery mildew resistance.

In one aspect, the invention provides a method for producing a modified wheat plant, comprising:
knocking down and/or knocking out *TaMLO-A1, TaMLO-B1* and/or *TaMLO-D1* genes in a wheat plant; and
increasing expression of TaTMT3 protein, preferably TaTMT3B protein, in the wheat plant, thereby obtaining a modified wheat plant which has increased resistance to powdery mildew and comparable or preferably increased yield relative to a corresponding wild-type wheat plant.

In one aspect, the invention further provides a method for producing a modified wheat plant, the method comprising:
providing a first wheat plant in which *TaMLO-A1, TaMLO-B1,* and *TaMLO-D1* gene is(are) knocked down and/or knocked out;
providing a second wheat plant in which expression of TaTMT3 protein, preferably TaTMT3B protein, is increased; and
crossing the first wheat plant with the second wheat plant to obtain a modified wheat plant in which *TaMLO-A1, TaMLO-B1* and/or *TaMLO-D1* genes is(are) knocked down and/or knocked out, and expression of TaTMT3 protein, preferably TaTMT3B protein, is increased.

In some embodiments, the modified wheat plant has increased resistance to powdery mildew and comparable or preferably increased yield relative to a corresponding wild-type wheat plant. In some embodiments, the first wheat plant has increased resistance to powdery mildew relative to a corresponding wild-type wheat plant. In some embodiments, the second plant has increased yield relative to a corresponding wild-type wheat plant.

In one aspect, the invention further provides a method for producing a modified wheat plant, comprising increasing expression of TaTMT3 protein, preferably TaTMT3B protein, in a wheat plant, thereby obtaining a modified wheat plant which has increased yield relative to a corresponding wild-type wheat plant. In some embodiments, the wheat plant has increased resistance to powdery mildew relative to the corresponding wild-type wheat plant.

In one aspect, the present invention further provides use of TaTMT3 protein, preferably TaTMT3B protein, or a nucleic acid molecule encoding the same, for the production of a wheat plant having increased yield relative to a corresponding wild-type wheat plant. In some embodiments, the wheat plant has increased resistance to powdery mildew relative to the corresponding wild-type wheat plant.

By "corresponding wild-type wheat plant" is meant an unmodified wheat plant from which the modified wheat plant is derived.

As used herein, "knock down" means that expression and/or activity of a gene of interest (typically an endogenous gene of interest) in a wheat plant is down-regulated by an artificial manipulation (e.g., a genetic manipulation) relative to a wild-type plant that has not received the manipulation. Expression may be the expression of the gene at the transcriptional or translational level. Knock down of the gene of interest can result in decreased function. Knock down of the gene of interest, for example, also encompasses mutation (e.g., point mutation) of its encoded product resulting in reduced activity, e.g., biological activity.

As used herein, "knock out" means that a gene of interest (typically an endogenous gene of interest) in a wheat plant is rendered substantially non-expressed, i.e., does not produce a functional expression product, and/or expresses a product that is substantially non-functional, by an artificial manipulation (e.g., a genetic manipulation), relative to a wild-type plant that has not received the manipulation. Expression may be the expression of the gene at the transcriptional or translational level. Knockout of the gene of interest can result in loss of function. Knock out of the gene of interest, for example, also encompasses mutation (e.g., point mutation) of its encoded product resulting in loss of activity, e.g., biological activity.

In some embodiments, *TaMLO-A1, TaMLO-B1,* and *TaMLO-D1* genes in the wheat plant are knocked out.

Several methods for knocking down or knocking out a gene of interest in plants are known in the art. Exemplary methods include, but are not limited to, introduction of antisense RNA, artificial miRNA (amiRNA), siRNA, shRNA targeting a gene of interest, or a gene editing system targeting a gene of interest such as a meganuclease, zinc finger nuclease (ZFN), transcription activator-like effector nuclease (TALEN), or CRISPR gene editing system, etc. into a plant.

The introduction may be achieved by transforming the plant with an expression construct comprising a nucleotide sequence encoding the antisense RNA, artificial miRNA (amiRNA), siRNA, shRNA, or components of the gene editing system. In the expression construct, the coding nucleotide sequence is typically operably linked to an expression regulatory element.

Introduction of antisense RNA, amiRNA, siRNA or shRNA targeting the gene of interest often results in degradation or translational repression of the transcription product of the gene of interest.

Introduction of a gene editing system targeting a gene of interest may result in a mutation in the gene of interest, such as a substitution, deletion or addition of one or more nucleotides, or even a partial or complete deletion of the gene, thereby resulting in that the gene of interest cannot transcribe or the gene of interest only produce a truncated or mutated protein with reduced or loss of function. The mutation caused by the gene editing system may be located in a regulatory sequence (e.g., promoter, enhancer, etc.) or a coding sequence of the gene of interest, and is preferably located in the coding sequence as long as the knock-down or knock-out of the gene of interest is achieved. For example, the mutation in the coding sequence is a frameshift mutation. Preferably, the mutation in the gene of interest is homozygous in the plant.

In some embodiments of the invention, *TaMLO-A1, TaMLO-B1* and/or *TaMLO-D1* genes in a wheat plant is(are) knocked out or knocked down by introducing antisense RNA, amiRNA, siRNA and shRNA.

In other embodiments of the invention, *TaMLO-A1, TaMLO-B1* and/or *TaMLO-D1* genes in a wheat plant is(are) knocked out or knocked down by introducing a gene editing system, such as a meganuclease, zinc finger nuclease, transcriptional activator-like effector nuclease (TALEN) or CRISPR gene editing system, resulting in mutation(s) in the *TaMLO-A1, TaMLO-B1* and/or *TaMLO-D1* genes. In some embodiments, the method further comprises a step of obtaining a wheat plant homozygous for the mutation(s) in the *TaMLO-A1, TaMLO-B1* and/or *TaMLO-D1* genes.

In some specific embodiments of the invention, *TaMLO-A1, TaMLO-B1* and/or *TaMLO-D1* genes in the wheat plant is(are) knocked out or knocked down by introducing a transcriptional activator-like effector nuclease (TALEN).

A "transcriptional activator-like effector nuclease (TALEN)" is a restriction enzyme that can be engineered to cleave a particular DNA sequence, typically prepared by fusing the DNA binding domain of a transcriptional activator-like effector TALE to a DNA cleavage domain. TALE can be engineered to bind almost any desired DNA sequence. The design and preparation of TALEN suitable for use in the invention can be found, for example, in Wang et al. (Nature Biotechnology, Volume 32, Number 9, September, 2014).

In other embodiments, *TaMLO-A1, TaMLO-B1* and/or *TaMLO-D1* genes in the wheat plant is(are) knocked out or knocked down by introducing a CRISPR gene editing system.

The CRISPR (Clustered regularly interspaced short palindromic repeats) system is an immune system developed by bacteria during evolution to defend against invasion by foreign genes, and has now been engineered and widely used for genome editing in eukaryotes.

CRISPR gene editing system usually comprises at least a CRISPR nuclease and a corresponding guide RNA (gRNA). gRNA comprises a targeting moiety having homology to a target nucleic acid sequence and a scaffold moiety responsible for binding to CRISPR nuclease. CRISPR nuclease, when complexed with the gRNA, is capable of targeting the target sequence on the genome under the direction of gRNA and performing its nucleic acid cleavage or other functions.

Nucleases that can be used by the CRISPR gene editing system include, but are not limited to, Cas3, Cas8a, Cas5, Cas8b, Cas8c, Cas10d, Cse1, Cse2, Csy1, Csy2, Csy3, GSU0054, Cas10, Csm2, Cmr5, Cas10, Csx11, Csx10, Csf1, Cas9, Csn2, Cas4, Cpf1, C2c1, C2c3 or C2c2 proteins or their variants or derivatives.

Based on the CRISPR system, a variety of gene editing systems have been developed in the art and are applicable in the invention.

For example, the CRISPR gene editing system also encompasses so-called base editing system, which is based on a fusion protein of a CRISPR nuclease (in particular CRISPR nuclease with nicking enzyme activity) and a cytosine deaminase or an adenine deaminase, enabling precise base substitutions in the genome.

Those skilled in the art are able to select, design and obtain various CRISPR gene editing systems suitable for the invention.

By "increasing expression of TaTMT3 protein in the wheat plant" is meant that the expression and/or the activity of the TaTMT3 protein in the wheat plant is up-regulated by an artificial manipulation (e.g., a genetic manipulation), relative to a corresponding wild-type plant which has not received the manipulation. "Increasing expression of TaTMT3 protein in the wheat plant" also encompasses introducing a mutation in TaTMT3 protein that increases its activity (e.g., biological activity), or introducing a TaTMT3 protein with increased activity (e.g., biological activity).

Several methods are known in the art to increase the expression of a given protein of interest in a plant. For example, in some embodiments, expression of TaTMT3 protein is increased by introducing an expression construct comprising a nucleotide sequence encoding the TaTMT3 protein into a wheat plant, wherein the nucleotide sequence encoding TaTMT3 protein is operably linked to an expression regulatory element. Preferably, the nucleotide sequence encoding TaTMT3 protein is operably linked to a strong promoter in the plant.

However, it is also possible to increase expression of TaTMT3 protein, preferably TaTMT3B protein, by engineering the endogenous *TaTMT3* gene, e.g., by modifying (e.g., using the gene editing system described in the invention) an expression regulation sequence, e.g., a promoter, of the endogenous *TaTMT3* gene to increase the expression of TaTMT3 protein, preferably TaTMT3B protein.

Methods suitable for introducing RNA molecules of the invention, such as antisense RNA, artificial miRNA (amiRNA), siRNA, shRNA, etc., components of gene editing system, or expression construct of TaTMT3 protein, preferably TaTMT3B protein, into a plant are known in the art and include, but are not limited to, protoplast electroporation, biolistic methods, PEG-mediated protoplast transformation, agrobacterium-mediated transformation.

In some embodiments, a nucleotide sequence encoding the RNA molecule, e.g., antisense RNA, artificial miRNA (amiRNA), siRNA, shRNA, etc., components of the genome editing system, or TaTMT3 protein, preferably TaTMT3B protein, is integrated into the plant genome for stable heritable expression. In some embodiments, especially for the genome editing system, it is transiently transformed into the plant. Transient transformation of the genome editing system results in heritable genetic modifications while the components thereof do not need to be integrated into the plant genome.

An exemplary coding sequence of the wild-type *TaMLO-A1* gene is shown in SEQ ID NO: 1 and the corresponding exemplary amino acid sequence is shown in SEQ ID NO: 2. An exemplary coding sequence of the wild-type *TaMLO-B1* gene is shown in SEQ ID NO: 3, and a corresponding exemplary amino acid sequence is shown in SEQ ID NO: 4. An exemplary coding sequence of the wild-type *TaMLO-D1* gene is shown in SEQ ID NO: 5, and a corresponding exemplary amino acid sequence is shown in SEQ ID NO: 6. Those skilled in the art would understand that for different wheat species, subspecies, cultivars or lines, their wild-type *MLO* gene may differ due to natural genetic polymorphisms, and therefore the sequences may differ from the exemplary sequences described above, but perform similar or the same functions in the plant. Such *MLO* sequences are also within the scope of the invention.

The TaTMT3 protein of the various aspects of the invention may be TaTMT3A, TaTMT3B and/or TaTMT3D protein, preferably TaTMT3B protein.

An exemplary coding sequence of TaTMT3B protein of the invention is shown in SEQ ID NO: 7, and a corresponding exemplary amino acid sequence is shown in SEQ ID NO: 8. An exemplary coding sequence of TaTMT3A protein of the invention is shown in SEQ ID NO: 9, and a corresponding exemplary amino acid sequence is shown in SEQ ID NO: 10. An exemplary coding sequence of TaTMT3D protein of the invention is shown in SEQ ID NO: 11, and a corresponding exemplary amino acid sequence is shown in SEQ ID NO: 12.

However, those skilled in the art can expect that some conservative substitutions in amino acid sequence or substitutions in non-critical domains, particularly differences due to natural genetic polymorphisms, will not substantially affect the function of proteins. Therefore, the invention also encompasses TaTMT3B proteins having at least 85%, 86%, 87%, 88%, 89%, 90%, 92%, 94%, 95%, 96%, 97%, 98% or 99% or more sequence identity to SEQ ID NO: 8, or having one or more (e.g., 1, 2, 3, 4, 5, 6, 7, 8, 9, 10) amino acid substitutions compared to SEQ ID NO: 8, which have similar or identical function to SEQ ID NO: 8. The invention also encompasses TaTMT3A proteins having at least 85%, 86%, 87%, 88%, 89%, 90%, 92%, 94%, 95%, 96%, 97%, 98% or 99% or more sequence identity to SEQ ID NO: 10, or having one or more (e.g. 1, 2, 3, 4, 5, 6, 7, 8, 9, 10) amino acid substitutions compared to SEQ ID NO: 10, which have similar or identical functions to SEQ ID NO: 10. The invention also encompasses TaTMT3D proteins having at least 85%, 86%, 87%, 88%, 89%, 90%, 92%, 94%, 95%, 96%, 97%, 98% or 99% or more sequence identity to SEQ ID NO: 12, or having one or more (e.g. 1, 2, 3, 4, 5, 6, 7, 8, 9, 10) amino acid substitutions compared to SEQ ID NO: 12, which have similar or identical functions to SEQ ID NO: 12.

In a further aspect, the invention provides a modified wheat plant, or progeny or parts thereof, wherein in the modified wheat plant, *TaMLO-A1, TaMLO-B1* and/or *TaMLO-D1* genes is(are) knocked down and/or knocked out, and expression of TaTMT3 protein, preferably TaTMT3B protein, is increased, and the modified wheat plant has increased resistance to powdery mildew and comparable or preferably increased yield relative to a wild-type wheat plant. In some embodiments, the modified wheat plant expresses (preferably constitutively expresses) an antisense RNA, amiRNA, siRNA or shRNA targeting *TaMLO-A1, TaMLO-B1* and/or *TaMLO-D1* genes, e.g., comprising a nucleotide sequence encoding the antisense RNA, amiRNA, siRNA or shRNA operably linked to an expression regulatory element integrated into its genome. In some embodiments, the modified wheat plant comprises a mutation in *TaMLO-A1, TaMLO-B1* and/or *TaMLO-D1* genes that results in the *TaMLO-A1, TaMLO-B1* and/or *TaMLO-D1* genes being knocked down and/or knocked out. In some embodiments, the modified wheat plant comprises a nucleotide sequence encoding a TaTMT3 protein, preferably TaTMT3B protein, operably linked to an expression regulatoryelement.

In another aspect, the invention provides a modified wheat plant or progeny or parts thereof, the modified wheat plant has increased expression of TaTMT3 protein, preferably TaTMT3B protein, and has increased yield relative to a wild-type wheat plant. In some embodiments, the modified wheat plant comprises a nucleotide sequence encoding a TaTMT3 protein, preferably TaTMT3B protein, operably linked to an expression regulatory element.

In another aspect, the invention provides a modified wheat plant or progeny or parts thereof, wherein the modified wheat plant can be produced or is produced by the method of the invention.

In some embodiments, the part of a wheat plant is a seed.

In a further aspect, the invention also provides use of the modified wheat plant of the invention or progeny or parts thereof in plant breeding. For example, the modified wheat plant or progeny or parts thereof may be used to incorporate other wheat traits of interest by conventional breeding or molecular breeding.

"Knock-down" or "knock-out" of *TaMLO-A1, TaMLO-B1* or *TaMLO-D1* gene, or an increase in TaTMT3 protein expression can be determined by detecting the amount of functional transcripts of the relevant gene (e.g., by quantifying RT-PCR) or the amount of functional proteins (e.g., by Western blotting) in the plant. For example, *TaMLO-A1* is considered to be knocked out if the presence of the transcript of *TaMLO-A1* in the plant is substantially undetectable by quantifying RT-PCR.

Resistance of wheat plants to powdery mildew can be determined by methods known in the art. For example, it can be evaluated by inoculating wheat leaves with pathogenic bacteria and observing the survival, growth or spot size produced by the pathogenic bacteria.

Yield of a wheat plant according to the invention preferably refers to the yield of wheat grains, which can be assessed, e.g., by the thousand grain weight of the grains. Yield can also refer to actual yield, e.g., acre yield. The yield of the wheat plant according to the invention can also be the biomass of wheat, which can be reflected, for example, by plant height.

In some embodiments, the modified wheat plant of the invention has a comparable yield relative to a corresponding wild-type plant, e.g., the yield of the modified wheat plant of the invention is not statistically significantly different from the yield of corresponding wild type plant, in particular in the absence of powdery mildew stress. In some preferred embodiments, the modified wheat plant of the invention has an increased yield relative to a corresponding wild-type plant, such as an increase in yield of about 5%, about 10%, about 15%, about 20%, about 25%, about 30%, about 35%, about 40%, about 50%, about 60%, about 70%, about 80%, about 90%, about 100%, about 150%, about 200% or more. In some embodiments, the increase in yield is an increase in the presence of powdery mildew stress. In some embodiments, the increase in yield is an increase in the absence of powdery mildew stress.

The wheat plant of the invention includes, but is not limited to, Triticum aestivum, T. aethiopicum, T. araraticum, T. boeoticum, T. carthlicum, T. compactum, T. dicoccoides, T. dicoccum, T. durum, T. ispahanicum, T. karamyschevii, T. macha, T. militinae, T. monococcum, T. polonicum, T. repens, T. spelta, T. sphaerococcum, T. timopheevii, T. turanicum, T. turgidum, T. urartu, T. vavilovii and T. zhukovskyi. In some preferred embodiments, the wheat plant belongs to Triticum aestivum, in particular Triticum aestivum cultivar Bobwhite.

The invention further provides reagents or kits for use in the methods of the invention, which may comprise antisense RNA, artificial miRNA (amiRNA), siRNA, shRNA of *TaMLO-A1, TaMLO-B1* and/or *TaMLO-D1* genes, or a gene editing system such as a meganuclease, zinc finger nuclease (ZFN), transcription activator-like effector nuclease (TALEN) or CRISPR gene editing system, or an expression construct encoding the same; and/or an expression construct encoding TaTMT3 protein, preferably TaTMT3B protein.

### Examples

The invention will now be described with reference to the following examples which are intended to illustrate, but not to limit, the invention.

### Example 1: Yield reduction of KN199 wheat mlo triple mutant plant

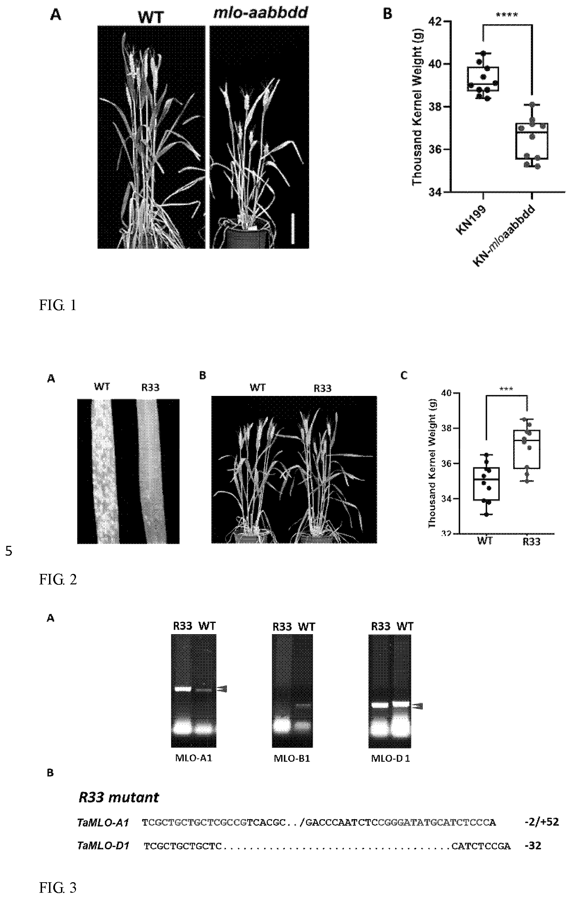

KN199 wheat *mlo* triple mutant plant (Wang et al., Nature Biotechnology, Volume 32, Number 9, September, 2014) previously obtained by the present inventors were grown and compared with wild type plants. The results showed that the mutant plants exhibit premature leaf senescence at the late growth stage, which results in decrease of yield (FIG. 1).

### Example 2: Mutant R33 has the phenotype of disease resistance and yield increase

TALEN vector was delivered into BW wheat variety by biolistic transformation technology. The TALEN vector simultaneously targets TaMLO-A1, TaMLO-B1 and TaMLO-D1 in the wheat genome (The specific information of TALEN vector can be found in Wang et al., Nature Biotechnology, Volume 32, Number 9, September, 2014). In a process of screening and detecting MLO1 gene-directed editing mutants, a new mutant R33 was found. The mutant and wild-type wheat were inoculated with powdery mildew respectively, and it was found that the mutant showed strong resistance to powdery mildew compared with the wild-type wheat. Meanwhile, the growth phenotypes of the mutant and the wild type were observed, and it was found that the mutant was not different from the wild type in the growth phenotype. Subsequently, 1000-grain weight of the mutant wheat was measured, and it was found that the 1000-grain weight of the mutant plant was significantly increased compared to the wild type. (FIG. 2).

### Example 3: Genotype identification of mutant R33

First, primers were designed at two ends of the target sites of TaMLO-A1, TaMLO-B1 and TaMLO-D1, respectively, and the genome DNA of the mutant plant was used as a template for PCR amplification to obtain the target fragment. Agarose gel electrophoresis results showed that the target band could be amplified on both A and D genomes. Sequencing analysis showed that -2/+52 mutation occurred in TaMLO-A1 and -32 mutation occurred in TaMLO-D1. However, the target band could not be amplified on the B genome (FIG. 3).

To explore the changes that occur in the B genome in mutant R33, a series of genetic experiments were further performed, including crossing between R33 and *mloaaBBdd,* WT and *mloaabbdd* (Table 1). Through these genetic analyses, mutations occurring in the B genome were considered to be recessive and near the TaMLO-B1 gene.

**Table 1. Crossing experiments of mutant R33 with other wheat genotypes**

| **Cross** | **Susceptible** | **Resistance** | **Total** | **Genotypes** |
|---|---|---|---|---|
| **R33 × double M (*mloaaB^{r}B^{r}dd*) (*mloaaBBdd*)** | **118** | **40** | **158** | ***aaBBdd, aaB*^{r}*Bdd, aaB^{r}B^{r}dd*** |
| **R33 × W'T (*mloaaB^{r}B^{r}dd*) (*mloAABBDD*)** | **97** | **4** | **101** | ***AABBDD, AABBDd, AABBdd, AaBBDD, AaBBDd, AaBBdd, aaBBDD, aaBBDd, aaBBdd, AABB^{r}DD, AABB^{r}Dd, AABBrdd AaBB^{r}DD, AaBB^{r}Dd, AaBBrdd, aaBB*^{r}*****DD aaBBrDd, aaBBrdd, AASrB^{r}DD, AAB^{r}B^{r}Dd AAB^{r}B^{r}dd, AaB^{r}B^{r}DD, AaB^{r}B^{r}Dd, AaB^{r}B^{r}dd, aaB^{r}B^{r}DD, aaB^{r}B^{r}Dd,** aaB^{r}B^{r}dd* |
| **R33 × triple M (*mloaaB^{r}B^{r}dd*) (*mloaabbdd*)** | **0** | **27** | **27** | ***aaB^{r}B^{r}dd, aaB^{r}bdd, aabbdd*** |

| | | | | |
|---|---|---|---|---|
| Assuming the genotype of mutant R33 is *mloaaB*^{r}B^{r}*dd* | | | | |

### Example 4: Resequencing analysis of mutant R33

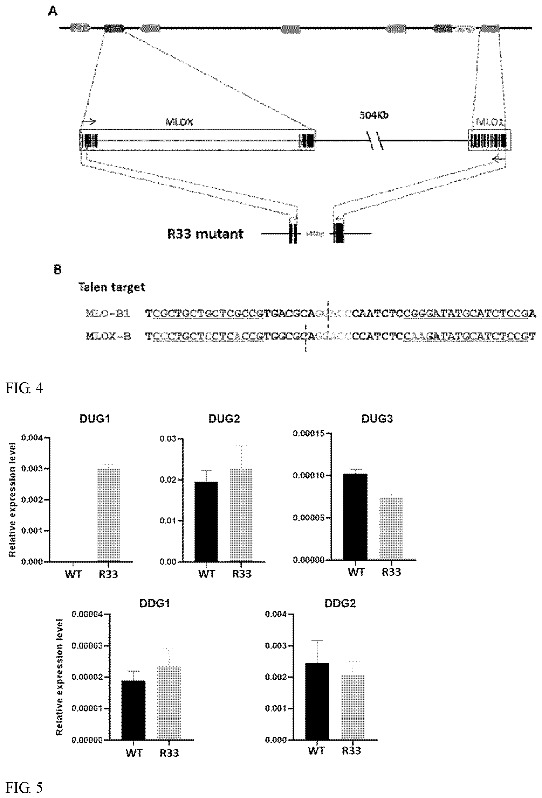

In order to identify the mutation pattern of the R33 mutant plant, NGS-based resequencing analysis was performed. Genetic changes occurring in R33 mutant plants were determined by alignment of the sequencing results with the target regions of the wild-type wheat reference genome: deletion of 304.374 KB occurred in the B genome of R33 mutant, from 612856038 bp to 613160412 bp, and a vector sequence, 344 bp, was inserted, as shown in FIG. 4. The upstream deletion site is located in a homologous gene of *MLO1 ,* named *MLOX.* The sequence around the cleavage site is highly homologous to the target sequence of TALEN on *MLO1* (FIG. 4).

### Example 5: Cause of the phenotype of mutant R33

In order to clarify the effect of fragment deletion on gene expression in R33 mutant, expression level of genes near the deleted fragment was detected. The results showed that the expression level of *DUG1* gene was significantly increased compared with the wild type (FIG. 5).

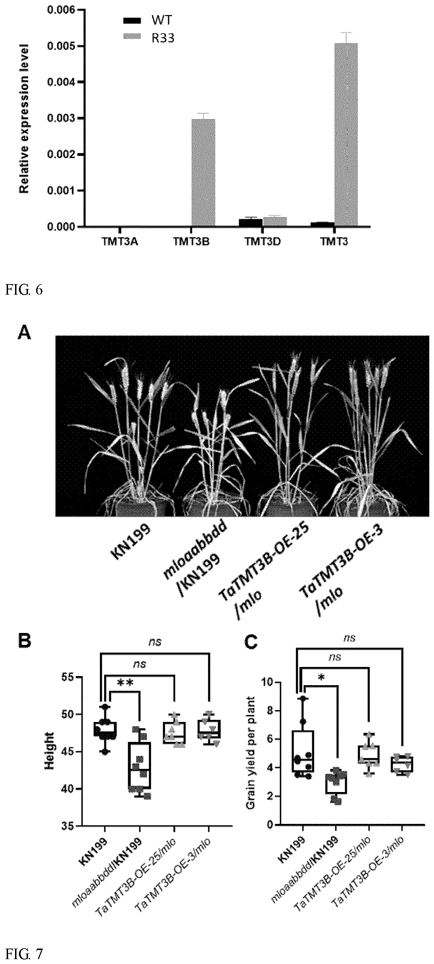

Further homology analysis of *DUG1* revealed that it encodes a vacuolar membrane monosaccharide transporter *TaTMT3.* We further examined the expression levels of *TaTMT3A, TaTMT3B* and *TaTMT3D,* and the results showed that only the expression level of *TaTMT3B* was significantly increased, while the expression levels of *TaTMT3A* and *TaTMT3B* were unchanged from the wild type. Universal primers for *TaTMT3A, TaTMT3B* and *TaTMT3D* were designed to detect the overall expression level of *TaTMT3,* and the results showed that the overall expression level of *TaTMT3* was significantly increased compared with the wild type (FIG. 6). Overexpression of *AtTMT1,* a homologous gene in *Arabidopsis thaliana,* has been reported to significantly increase yield.

In conclusion, gene editing resulted in loss-of-function mutations of *TaMLO-A1* and *TaMLO-D1* in the R33 mutant, as well as deletion of large fragments near *TaMLO-B1* in the B genome, which eventually led to the loss of function of *TaMLO-A1, TaMLO-B1* and *TaMLO-D1* proteins, as well as increased expression of TaTMT3B, thus making the R33 mutant resistant to powdery mildew and have increased yield. The invention provides a method that balances powdery mildew resistance and growth defects in wheat.

**Example 6: Overexpression of TaTMT3B improves the phenotype of *mlo-aabbdd* triple mutant wheat**

To further verify the biological function of TaTMT3, *TaTMT3B* was overexpressed in the background of *mloaabbdd* triple mutant wheat. It was found that overexpression of TaTMT3B restored the premature senescence phenotype caused by mutation of the MLO genes (FIG. 7A). At the same time, plant height and yield were also restored to wild-type levels (FIG. 7B and FIG. 7C).

### Sequence Listing:

SEQ ID NO: 1 coding nucleotide sequence of TaMLO-A1 
ATGGCAAAGGACGACGGGTACCCCCCGGCGCGGACGCTGCCGGAGACGCCGTCCTGGGCGGTGGCG
CTGGTCTTCGCCGTCATGATCATCGTCTCCGTCCTCCTGGAGCACGCGCTCCACAAGCTCGGCCATTGG
TTCCACAAGCGGCACAAGAACGCGCTGGCGGAGGCGCTGGAGAAGATGAAGGCGGAGCTGATGCTG
GTGGGATTCATCTCGCTGCTGCTCGCCGTCACGCAGGACCCAATCTCCGGGATATGCATCTCCCAGAAG
GCCGCCAGCATCATGCGCCCCTGCAAGGTGGAACCCGGTTCCGTCAAGAGCAAGTACAAGGACTACT
ACTGCGCCAAAGAGGGCAAGGTGGCGCTCATGTCCACGGGCAGCCTGCACCAGCTCCACATATTCATC
TTCGTGCTAGCCGTCTTCCATGTCACCTACAGCGTCATCATCATGGCTCTAAGCCGTCTCAAGATGAGA
ACATGGAAGAAATGGGAGACAGAGACCGCCTCCTTGGAATACCAGTTCGCAAATGATCCTGCGCGGTT
CCGCTTCACGCACCAGACGTCGTTCGTGAAGCGGCACCTGGGCCTGTCCAGCACCCCCGGCGTCAGA
TGGGTGGTGGCCTTCTTCAGGCAGTTCTTCAGGTCGGTCACCAAGGTGGACTACCTCACCTTGAGGGC
AGGCTTCATCAACGCGCACTTGTCGCAGAACAGCAAGTTCGACTTCCACAAGTACATCAAGAGGTCCA
TGGAGGACGACTTCAAAGTCGTCGTTGGCATCAGCCTCCCGCTGTGGGCTGTGGCGATCCTCACCCTC
TTCCTTGATATCGACGGGATCGGCACACTCACCTGGGTTTCTTTCATCCCTCTCATCATCCTCTTGTGTG
TTGGAACCAAGCTAGAGATGATCATCATGGAGATGGCCCTGGAGATCCAGGACCGGTCGAGCGTCATC
AAGGGGGCACCCGTGGTCGAGCCCAGCAACAAGTTCTTCTGGTTCCACCGCCCCGACTGGGTCCTCT
TCTTCATACACCTGACGCTGTTCCAGAACGCGTTTCAGATGGCACATTTCGTGTGGACAGTGGCCACG
CCCGGCTTGAAGGACTGCTTCCATATGAACATCGGGCTGAGCATCATGAAGGTCGTGCTGGGGCTGGC
TCTCCAGTTCCTGTGCAGCTACATCACCTTCCCCCTCTACGCGCTAGTCACACAGATGGGATCAAACAT
GAAGAGGTCCATCTTCGACGAGCAGACAGCCAAGGCGCTGACCAACTGGCGGAACACGGCCAAGGA
GAAGAAGAAGGTCCGAGACACGGACATGCTGATGGCGCAGATGATCGGCGACGCAACACCCAGCCG
AGGCACGTCCCCGATGCCTAGCCGGGGCTCATCGCCGGTGCACCTGCTTCAGAAGGGCATGGGACGG
TCTGACGATCCCCAGAGCGCACCGACCTCGCCAAGGACCATGGAGGAGGCTAGGGACATGTACCCGG
TTGTGGTGGCGCATCCTGTACACAGACTAAATCCTGCTGACAGGCGGAGGTCGGTCTCTTCATCAGCC
CTCGATGCCGACATCCCCAGCGCAGATTTTTCCTTCAGCCAGGGATGA
SEQ ID NO: 2 amino acid sequence of TaMLO-A1 
MAKDDGYPPARTLPETPSWAVALVFAVMIIVSVLLEHALHKLGHWFHKRHKNALAEALEKMKAELMLVG
FISLLLAVTQDPISGICISQKAASIMRPCKVEPGSVKSKYKDYYCAKEGKVALMSTGSLHQLHIFIFVLAVFH
VTYSVIIMALSRLKMRTWKKWETETASLEYQFANDPARFRFTHQTSFVKRHLGLSSTPGVRWVVAFFRQF
FRSVTKVDYLTLRAGFINAHLSQNSKFDFHKYIKRSMEDDFKVVVGISLPLWAVAILTLFLDIDGIGTLTWV
SFIPLIILLCVGTKLE
MIIMEMALEIQDRSSVIKGAPVVEPSNKFFWFHRPDWVLFFIHLTLFQNAFQMAHFVWTVATPGLKDCFH
MNIGLSIMKVVLGLALQFLCSYITFPLYALVTQMGSNMKRSIFDEQTAKALTNWRNTAKEKKKVRDTDML
MAQMIGDATPSRGTSPMPSRGSSPVHLLQKGMGRSDDPQSAPTSPRTMEEARDMYPVVVAHPVHRLNPA
DRRRSVSSSALDADIPSADFSFSQG
SEQ ID NO: 3 coding nucleotide sequence of TaMLO-B1 
ATGGCGGACGACGACGAGTACCCCCCAGCGAGGACGCTGCCGGAGACGCCGTCCTGGGCGGTGGCC
CTCGTCTTCGCCGTCATGATCATCGTGTCCGTCCTCCTGGAGCACGCGCTCCATAAGCTCGGCCATTGG
TTCCACAAGCGGCACAAGAACGCGCTGGCGGAGGCGCTGGAGAAGATCAAGGCGGAGCTCATGCTG
GTGGGCTTCATCTCGCTGCTGCTCGCCGTGACGCAGGACCCCATCTCCGGGATATGCATCTCCGAGAA
GGCCGCCAGCATCATGCGGCCCTGCAAGCTGCCCCCTGGCTCCGTCAAGAGCAAGTACAAAGACTACT
ACTGCGCCAAACAGGGCAAGGTGTCGCTCATGTCCACGGGCAGCTTGCACCAGCTGCACATATTCATC
TTCGTGCTCGCCGTCTTCCATGTCACCTACAGCGTCATCATCATGGCTCTAAGCCGTCTCAAAATGAGA
ACCTGGAAGAAATGGGAGACAGAGACCGCCTCCCTGGAATACCAGTTCGCAAATGATCCTGCGCGGT
TCCGCTTCACGCACCAGACGTCGTTCGTGAAGCGGCACCTGGGCCTCTCCAGCACCCCCGGCGTCAG
ATGGGTGGTGGCCTTCTTCAGGCAGTTCTTCAGGTCGGTCACCAAGGTGGACTACCTCACCTTGAGGG
CAGGCTTCATCAACGCGCATTTGTCGCATAACAGCAAGTTCGACTTCCACAAGTACATCAAGAGGTCC
ATGGAGGACGACTTCAAAGTCGTCGTTGGCATCAGCCTCCCGCTGTGGTGTGTGGCGATCCTCACCCT
CTTCCTTGACATTGACGGGATCGGCACGCTCACCTGGATTTCTTTCATCCCTCTCGTCATCCTCTTGTGT
GTTGGAACCAAGCTGGAGATGATCATCATGGAGATGGCCCTGGAGATCCAGGACCGGGCGAGCGTCAT
CAAGGGGGCGCCCGTGGTTGAGCCCAGCAACAAGTTCTTCTGGTTCCACCGCCCCGACTGGGTCCTC
TTCTTCATACACCTGACGCTATTCCAGAACGCGTTTCAGATGGCACATTTCGTGTGGACAGTGGCCACG
CCCGGCTTGAAGAAATGCTTCCATATGCACATCGGGCTGAGCATCATGAAGGTCGTGCTGGGGCTGGC
TCTTCAGTTCCTCTGCAGCTATATCACCTTCCCGCTCTACGCGCTCGTCACACAGATGGGATCAAACAT
GAAGAGGTCCATCTTCGACGAGCAGACGGCCAAGGCGCTGACAAACTGGCGGAACACGGCCAAGGA
GAAGAAGAAGGTCCGAGACACGGACATGCTGATGGCGCAGATGATCGGCGACGCGACGCCCAGCCG
AGGGGCGTCGCCCATGCCTAGCCGGGGCTCGTCGCCAGTGCACCTGCTTCACAAGGGCATGGGACGG
TCCGACGATCCCCAGAGCACGCCAACCTCGCCAAGGGCCATGGAGGAGGCTAGGGACATGTACCCGG
TTGTGGTGGCGCATCCAGTGCACAGACTAAATCCTGCTGACAGGAGAAGGTCGGTCTCGTCGTCGGC
ACTCGATGTCGACATTCCCAGCGCAGATTTTTCCTTCAGCCAGGGATGA
SEQ ID NO: 4 amino acid sequence of TaMLO-B1 
MADDDEYPPARTLPETPSWAVALVFAVMIIVSVLLEHALHKLGHWFHKRHKNALAEALEKIKAELMLVGFI
SLLLAVTQDPISGICISEKAASIMRPCKLPPGSVKSKYKDYYCAKQGKVSLMSTGSLHQLHIFIFVLAVFHV
TYSVIIMALSRLKMRTWKKWETETASLEYQFANDPARFRFTHQTSFVKRHLGLSSTPGVRWVVAFFRQFF
RSVTKVDYLTLRAGFINAHLSHNSKFDFHKYIKRSMEDDFKVVVGISLPLWCVAILTLFLDIDGIGTLTWISF
IPLVILLCVGTKLEMIIMEMALEIQDRASVIKGAPVVEPSNKFFWFHRPDWVLFFIHLTLFQNAFQMAHFV
WTVATPGLKKCFHMHIGLSIMKVVLGLALQFLCSYITFPLYALVTQMGSNMKRSIFDEQTAKALTNWRNT
AKEKKKVRDTDMLMAQMIGDATPSRGASPMPSRGSSPVHLLHKGMGRSDDPQSTPTSPRAMEEARDMY
PVVVAHPVHRLNPADRRRSVSSSALDVDIPSADFSFSQG
SEQ ID NO: Scoding nucleotide sequence of TaMLO-D1 
ATGGCGGAGGACTACGAGTACCCCCCGGCGCGGACGCTGCCGGAGACGCCGTCCTGGGCGGTGGCGC
TCGTCTTCGCCGTCATGATCATCGTGTCCGTCCTCCTGGAGCACGCGCTCCACAAGCTCGGCCATTGGT
TCCACAAGCGGCACAAGAACGCGCTGGCGGAGGCGCTGGAGAAGATCAAAGCGGAGCTGATGCTGG
TGGGGTTCATCTCGCTGCTGCTCGCCGTGACGCAGGACCCAATCTCCGGGATATGCATCTCCGAGAAG
GCCGCCAGCATCATGCGGCCCTGCAGCCTGCCCCCTGGTTCCGTCAAGAGCAAGTACAAAGACTACTA
CTGCGCCAAAAAGGGCAAGGTGTCGCTAATGTCCACGGGCAGCTTGCACCAGCTCCACATATTCATCT
TCGTGCTCGCCGTCTTCCATGTCACCTACAGCGTCATCATCATGGCTCTAAGCCGTCTCAAAATGAGGA
CATGGAAGAAATGGGAGACAGAGACCGCCTCCTTGGAATACCAGTTCGCAAATGATCCTGCGCGGTTC
 
CGCTTCACGCACCAGACGTCGTTCGTGAAGCGTCACCTGGGCCTCTCCAGCACCCCCGGCATCAGATG
GGTGGTGGCCTTCTTCAGGCAGTTCTTCAGGTCGGTCACCAAGGTGGACTACCTCACCCTGAGGGCA
GGCTTCATCAACGCGCATTTGTCGCATAACAGCAAGTTCGACTTCCACAAGTACATCAAGAGGTCCAT
GGAGGACGACTTCAAAGTCGTCGTTGGCATCAGCCTCCCGCTGTGGTGTGTGGCGATCCTCACCCTCT
TCCTTGATATTGACGGGATCGGCACGCTCACCTGGATTTCTTTCATCCCTCTCGTCATCCTCTTGTGTGT
TGGAACCAAGCTGGAGATGATCATCATGGAGATGGCCCTGGAGATCCAGGACCGGGCGAGCGTCATC
AAGGGGGCGCCCGTGGTTGAGCCCAGCAACAAGTTCTTCTGGTTCCACCGCCCCGACTGGGTCCTCTT
CTTCATACACCTGACGCTGTTCCAGAATGCGTTTCAGATGGCACATTTCGTCTGGACAGTGGCCACGCC
CGGCTTGAAGAAATGCTTCCATATGCACATCGGTCTGAGCATCATGAAGGTCGTGCTGGGGCTGGCTC
TTCAGTTCCTCTGCAGCTATATCACCTTCCCCCTCTACGCGCTCGTCACACAGATGGGATCGAACATGA
AGAGGTCCATCTTCGACGAGCAGACGGCCAAGGCGCTGACCAACTGGCGGAACACGGCCAAGGAGA
AGAAGAAGGTCCGAGACACGGACATGCTGATGGCGCAGATGATCGGCGACGCGACGCCCAGCCGAG
GCACGTCGCCGATGCCTAGCCGGGCTTCGTCACCGGTGCACCTGCTTCACAAGGGCATGGGACGGTCC
GACGATCCCCAGAGCGCGCCGACCTCGCCAAGGACCATGGAGGAGGCTAGGGACATGTACCCGGTTG
TGGTGGCGCATCCCGTGCACAGACTAAATCCTGCTGACAGGCGGAGGTCGGTCTCTTCGTCGGCACTC
GATGCCGACATCCCCAGCGCAGATTTTTCCTTCAGCCAGGGATGA
SEQ ID NO: 6 amino acid sequence of TaMLO-D1 
MAEDYEYPPARTLPETPSWAVALVFAVMIIVSVLLEHALHKLGHWFHKRHKNALAEALEKIKAELMLVGFI
SLLLAVTQDPISGICISEKAASIMRPCSLPPGSVKSKYKDYYCAKKGKVSLMSTGSLHQLHIFIFVLAVFHVT
YSVIIMALSRLKMRTWKKWETETASLEYQFANDPARFRFTHQTSFVKRHLGLSSTPGIRWVVAFFRQFFRS
VTKVDYLTLRAGFINAHLSHNSKFDFHKYIKRSMEDDFKVVVGISLPLWCVAILTLFLDIDGIGTLTWISFIP
LVILLCVGTKLEMIIMEMALEIQDRASVIKGAPVVEPSNKFFWFHRPDWVLFFIHLTLFQNAFQMAHFVWT
VATPGLKKCFHMHIGLSIMKVVLGLALQFLCSYITFPLYALVTQMGSNMKRSIFDEQTAKALTNWRNTAKE
KKKVRDTDMLMAQMIGDATPSRGTSPMPSRASSPVHLLHKGMGRSDDPQSAPTSPRTMEEARDMYPVV
VAHPVHRLNPADRRRSVSSSALDADIPSADFSFSQG
SEQ ID NO: 7 coding nucleotide sequence of TMT3B 
ATGGAAGATTTAACACGGCCTCTTGTGAGAGAGTCGGAGGCTAATCTTCATGCGAAGCTCGCTCCCTC
CGGCTCCGGATCTGGACTACGACAAGCGTGTAAGAAGTTGCGTGATTTGACGACCGTTGATCGCGCGG
TTCTTGTCGCGTTCGTGGCATCCATCGGCAACCTGCTCCAAGGCTGGGACAATGCCAGCATTGCAGGT
GCTATGTTTTACATAAAGGACGAGTTTAAACTAGATAGCATGCCAATGATTGAGGGGTGTATTATGGCTA
TGGCACTTTTTGGGGCAACAATAATCACAACACTATCTGGAATGCTATCTGATAAATTTGGTCGGTGGG
CGATGCTACTTACCTCGGCGGTATTGTCCTTTGTTAGTGCACTATTGGTCATATTTTGGTCCCAACATGT
GTATATGCTGCTATTCGTGAGGCTTATTCAGGGATTTAGTATTGGTTTGGCAGTTACACTTGTGCCATTAT
ATATAGTTGAGACTGCGCCTCCTGATATGAGAGGAAAATTAAGCACATTTCCCCAGTTAAGTGGTTCAG
CTGGTATGTTCTTATCTTACTGCATGGTGTTTTGGATGTCCATGCTGCCAAAGGTTAGTTGGCGGATCAT
GCTTGGGATACAACTGATACCCTCACTAATATACTCAATTTTAACTATCTTCTATCTACCTGAGACTCCGA
GTTGGCTTGTGAGCCAAGGAAGGGTGGATGAAGCCAAGATGGTTTTACAAAAACTACGAAGAAAGGA
AGATGTCTCAGGTGAAATGGCAAGTCTTTTGGAGGGCACACAAGTTGGTGACACTCCGTCTATTGAGG
AGTACCTAATTAGCACTAACGAAAGTATGTTAAGGGAAAAGGTGATTGGCAATGATGAAATAATAAAAT
TATATGGACTTCCAGAAGATTTGCATTGTGTTGCCTATCCATTGAAGAGAACAAACACAGAAGAAAGT
GCCATTGGTCATTCTGTGAGTCGTGGAGCATCGTTCTATGACCCAGTTGTCAACATTATTGGGAGTATG
CACGGGCTCCCTGAGGTTGCCCACGGCATATTCAATGAATTAGAACAACAAGGTCCAATTGAAGGGGA
 
TGAAGAGAATCAGGAAGAGACCAAAGAGCATGAGCTAGAACACAACCGAGATGATACCTATGATAGC
GAGCATGATTATCTTATTCAACCCAAACCTACGAATATAAATGATTTTGTGATATGTCGCAAAAGCGGCC
ATATTGGTGGAGGTTGGCAATTGGCTTGGAAAATGTCATCAGGATATCATTTGGATGGGCAAATGGAAG
GTGGCATGGAACGAGTATATTTGCATGAAGGAGGTGTACCAAGTTCTGAAAATCTACTGGATGCTCCAA
TAGACGGGAATTTCATTCAAGCGACTGCTTTGGTCAACAAATCGGTTTTTCACAAGTCTGGACACAATA
TTGGTATTCATTCACCTAATAAAGATTATAGAAGTACAAAATGGAAGGATCTGTTAGAACCTGGTGTAA
AACGTGCATTGGTCGTGGGTGTTGGAATACAAGTACTTCAACAGTTTGCTGGCATCAATGGCATTCTTT
ATTACACTCCTCAAATACTTGAGCAAGCTGGTGTTGGGGTTCTTCTCTCAAAGTTTGGCATCAACTCTT
CTTCAGTGTCGATTCTTATGAGCGCTCTCACAACTTTATTGATGCTTCCTTTTATCTGCATAGCCATGTGG
CTTATGGACCGCTCTGGAAGAAGGATACTACTTGTGACAATACCCATCTTGGTAGTGTCCCTCGTT
GTTTTAGTAACCGTCAACATTGTGAATCTGAGTGCTGAACTACACGCACTGCTCTCAACAATGAGTGTT
GGAATCTATTTCTGCATATTTGTCATGGGTTTTGGTCCAATACCAAATATATTCTGCTCAGAGATTTTCCC
CAACAAAGTTCGTGCTATTTGCTTGGCCATATGTAGCCTAGTCTTTTGGATTTGTGACATCATTGTGACA
TATACCCTTCCTGTATTGTTGAGGTATATAGGTCTTGCGGGTGTTTTCGGAGTTTATGCCATTGTTTGTGT
GTTGGCTTTTGTTTTCGTTTGTCTAAAGGTCCCGGAAACAAAGAATATACCTATTGAGGTCATAGCAGA
GTTCTATGCACTTGGCGGGTCAGGTACCCAAATCATCCAAGAGAGGCAGAAAGAAAATTCAGAGAAA
TTATTAGTGTGA
SEQ ID NO: 8 amino acid sequence of TMT3B 
MEDLTRPLVRESEANLHAKLAPSGSGSGLRQACKKLRDLTTVDRAVLVAFVASIGNLLQGWDNASIAGAM
FYIKDEFKLDSMPMIEGCIMAMALFGATIITTLSGMLSDKFGRWAMLLTSAVLSFVSALLVIFWSQHVYML
LFVRLIQGFSIGLAVTLVPLYIVETAPPDMRGKLSTFPQLSGSAGMFLSYCMVFWMSMLPKVSWRIMLGIQ
LIPSLIYSILTIFYLPETPSWLVSQGRVDEAKMVLQKLRRKEDVSGEMASLLEGTQVGDTPSIEEYLISTNES
MLREKVIGNDEIIKLYGLPEDLHCVAYPLKRTNTEESAIGHSVSRGASFYDPVVNIIGSMHGLPEVAHGIFNE
LEQQGPIEGDEENQEETKEHELEHNRDDTYDSEHDYLIQPKPTNINDFVICRKSGHIGGGWQLAWKMSSG
YHLDGQMEGGMERVYLHEGGVPSSENLLDAPIDGNFIQATALVNKSVFHKSGHNIGIHSPNKDYRSTKWK
DLLEPGVKRALVVGVGIQVLQQFAGINGILYYTPQILEQAGVGVLLSKFGINSSSVSILMSALTTLLMLPFIC
IAMWLMDRSGRRRILLVTIPILVVSLVVLVTVNIVNLSAELHALLSTMSVGIYFCIFVMGFGPIPNIFCSEIFP
NKVRAICLAICSLVFWICDIIVTYTLPVLLRYIGLAGVFGVYAIVCVLAFVFVCLKVPETKNIPIEVIAEFYAL
GGSGTQIIQERQKENSEKLLV
SEQ ID NO: 9 coding nucleotide sequence of TMT3A 
ATGGAAGATTTATCACGCCCTCTTGTGAGAGACTCGGAGGCTAATCTTCATGCCAAGCTCGCTCCCTCT
GGCCACGGATCTGGACTACTACAACAAGCGTGTCAGAAGTTGCGTGACTTGACGACCGTTGATCGCGC
GGTCCTCGTCGCGTTCGTGGCATCCATCGGCAACCTGCTCCAAGGCTGGGACAATGCGAGCATTGCAG
GTGCTATGCTTTACATAAAGGATGAGTTTAAACTAGATAGCATGCCAATGATTGAGGGGTGCATTATGGC
TATGGCACTTTTTGGGGCAACAATAATCACAACACTATCTGGAATGCTATCTAATAAATTTGGTAGGTGG
GCGATGCTACTTACCTCGGCGGTATTGTCCTTTGTTAGTGCACTATTGGTCATATTTTGGTCCCAACATG
TGTATATGCTGCTATTCGCGAGGCTTATTCAGGGATTTAGTATTGGTTTGGCAGTTACACTTGTGCCATTA
TATATAGTTGAGACCGCACCTCCTGATATGAGAGGAAAATTAAGCACATTTCCCCAGTTAAGTGGTTCA
GTTGGTATGTTCTTATCTTACTGCATGGTGTTTTGGATGTCCATGCTGCCAAAGGTTAGTTGGCGGGTCA
TGCTTGGGATACAACTGATACCCTCACTAATATACTCAATTTTAACAATCTTCTATCTACCCGAGACTCC
GAGTTGGCTTGTGAGCCAAGGAAGGGTGGATGAAGCAAAGATGGTTTTACAAAAACTACGAAGAAAG
GAAGATGTCTCAGGTGAAATGGCAAGTCTTTTGGAGGGCACACAAGTTGGTGACACTCCGTCTATTGA
 
GGAGTACCTAATTAGCACTAATGAAAGTATGTTAAGGGAAAAAGTGATTGGCAATGATGAAATAATAAA
ATTATATGGACTTCCAGAAGATTTGCATTGTGTTGCCTATCCATTGAAGAGAACAAACACAGAAGAAA
GTGCCATTGGTCATTCTGTGAGTCATGGAGCATCGTTCTATGACCCAGTTGTCAACATTATTGGGAGTAT
GCACGGGCTCCCCGAGGTTGCCCACGGCATATTCAATGAATTAGAACAACAAGGTCCAATTGAAGGGG
ACGAAGAGAATCAGGAAGAGACCAAAGAGCATGAGCTAGAACACAACCGAGATGGTACCTATGATAG
CGAGCATGCTTATCTTATTCAACCCAAACCTACGAATATAAATGATTTTGTGGTATGTCGCAAAAGCGGC
CATATTGGTGGAGGTTGGCAATTGGCTTGGAAAATGTCATCAGGATATCGTTTAGATGGGCAAATGGAA
GGTGGCATGGAACGAGTATATTTGCATGAAGGAGGTGTACCAAGTTCTGAAAACCTACTGGATGCTCC
AATAGATGGGAATTTCATTCAAGCGACTGCTTTGGTCAATAAATCGGTTTTTCATAAGTCTGGACACAAT
ATTGGTATTCATTCACCTAACAAAGATTATAGAAGTACAAAATGGAAGGATCTGTTAGAACCTGGTGTA
AAACGTGCATTGGTCGTGGGTGTTGGAATACAAGTGCTTCAACAGTTTGCTGGCATCAATGGCATTCTT
TATTACACTCCTCAAATACTTGAGCAAGCTGGTATTGGGGTTCTTCTCTCAAAGTTTGGCATCAGCTCTT
CTTCAGTGTCGATTCTTATGAGCGCTCTCACAACTTTATTGATGCTTCCTTTTATTTGCATGGCCATGTGG
CTTATGGACCGCTCTGGAAGAAGAAGGATACTACTTGTGACAATACCCATCTTGGTAGTGTCCCTCATT
GTTTTGGTAACCGTCAACATTGTGAATCTGAGTGCTGAACTACACGCACTGCTCTCAACAATGAGTGTT
GGAATCTATTTCTGCATATTTGTCATGGGTTTTGGTCCAATACCAAATATATTCTGCTCGGAGATTTTCCC
CAACAAAGTTCGTGCTATTTGCTTGGCCATATGTAGCCTAATCTTTTGGATTTGTGACATCATTGTGACA
TATACCCTCCCTGTATTGTTGAGGTATATAGGTCTTGCGGGTGTTTTCGGAGTTTATGCCATTGTTTGTGT
GCTGGCTTTTGTTTTCGTTTGTCTAAAGGTCCCGGAAACAAAGAATATACCTATTGAGGTCATAGAAGA
GTTCTATGCACTTGGCGGGTCAGACACCCAAATCATCCAAGAGAGGAAGAAAGAAAATTCAGAGAAA
TTATTAGTGTGA
SEQ ID NO: 10 amino acid sequence of TMT3A 
MEDLSRPLVRDSEANLHAKLAPSGHGSGLLQQACQKLRDLTTVDRAVLVAFVASIGNLLQGWDNASIAGA
MLYIKDEFKLDSMPMIEGCIMAMALFGATIITTLSGMLSNKFGRWAMLLTSAVLSFVSALLVIFWSQHVYM
LLFARLIQGFSIGLAVTLVPLYIVETAPPDMRGKLSTFPQLSGSVGMFLSYCMVFWMSMLPKVSWRVMLGI
QLIPSLIYSILTIFYLPETPSWLVSQGRVDEAKMVLQKLRRKEDVSGEMASLLEGTQVGDTPSIEEYLISTNE
SMLREKVIGNDEIIKLYGLPEDLHCVAYPLKRTNTEESAIGHSVSHGASFYDPVVNIIGSMHGLPEVAHGIFN
ELEQQGPIEGDEENQEETKEHELEHNRDGTYDSEHAYLIQPKPTNINDFVVCRKSGHIGGGWQLAWKMSS
GYRLDGQMEGGMERVYLHEGGVPSSENLLDAPIDGNFIQATALVNKSVFHKSGHNIGIHSPNKDYRSTKW
KDLLEPGVKRALVVGVGIQVLQQFAGINGILYYTPQILEQAGIGVLLSKFGISSSSVSILMSALTTLLMLPFIC
MAMWLMDRSGRRRILLVTIPILVVSLIVLVTVNIVNLSAELHALLSTMSVGIYFCIFVMGFGPIPNIFCSEIFP
NKVRAICLAICSLIFWICDIIVTYTLPVLLRYIGLAGVFGVYAIVCVLAFVFVCLKVPETKNIPIEVIEEFYALG
GSDTQIIQERKKENSEKLLV
SEQ ID NO: 11 coding nucleotide sequence ofTMT3D 
ATGGAAGATTTAACGCGTCCTCTTGTGAGAGATTCGGAGGCTAATCTTCATGCCAAGCTCGCTCCCTCC
GGCTCCGGATCTGGACTGCAACAAGCGTGTAAGAAGTTGCGTGATTTGACGACCGTTGATCGCGCGGT
TCTTGTCGCGTTCGTGGCATCCATCGGCAACCTGCTCCAAGGCTGGGACAATGCGAGCATTGCAGGTG
CTATGTTTTACATAAAGGATGAGTTTAAACTAGATAGCATGCTAATGATTGAGGGGTGTATTATGGCCAT
GGCACTTTTTGGGGCAACAATAATCACAACACTATCTGGAATGCTATCTGATAAATTTGGTAGGTGGGC
GATGCTACTTACCTCGGCGGTGTTGTCCTTTGTTAGTGCAGTATTGGTCATATTTTGGTCCCAACATGTG
TATATGCTGCTATTCGCGAGGCTTATTCAGGGATTTAGCATTGGTTTGGCAGTTACACTTGTGCCATTATA
TATAGTTGAGACCGCGCCTCCTGATATGAGAGGAAAATTAAGCACATTTCCCCAGTTAAGTGGTTCAGT
 
TGGTATGTTCTTGTCTTACTGCATGGTGTTTTGGATGTCCATGCTGCCAAAGGTTAGTTGGCGGATCATG
CTTGGGATACAACTGATACCCTCACTAATATACTCAATTTTAACTATCTTCTATCTACCCGAGACTCCGA
GTTGGCTTGTGAGCCAAGGAAGGGTGGATGAAGCCAAGATGGTTTTACAAAAACTACGAAGAAAGGA
AGATGTCTCAGGTGAAATGGCAAGTCTTTTGGAGGGTACACAAGTTGGTGATACTCCGTCTATTGAGG
AGTACCTAATTAGCACTAATGAAAGTATGTTAAGGGAAAAAGTGATTGGCAATGATGAAATAATAAAAT
TATATGGACTTCCGGAAGATTTGCATTGTGTTGCCTATCCATTGAAGAGAACAAACACAGAAGAAAGT
GCCATTGGTCATTCTGTGAGTCGTGGAGCATCGTTCTATGACCCAGTTGTCAACATTATTGGGAGTATG
CACGGGCTCCCTGAGGTTGCCCACGGCATATTCAATGAATTAGAACAACAAGGTCCAATTGAAGGGGA
CGAAGAGAATCAGGAAGAGACCAAAGAGCATGAGCTAGAACACAACCGAGATGATACCTATGATAGC
GAGCATGATTATCTTATTCAACCCAAACCTACGAATATAAATGATTTTGTTGTATGTCGCAAAAGCGGCC
ATATTGGTGGAGGTTGGCAATTGGCTTGGAAAATGTCATCAGGATATCATTTGGATGGGCAAATGGAAG
GTGGCATGGAACGGGTATATTTGCATGAAGGAGGTGTACCAAGTTCTGAAAACCTACTAGATGCTCCA
ATAGACGGGAATTTCATTCAAGCGACTGCTTTGGTCAACAAATCGGTTTTTCATAAGTCTGGACACAAT
ATTGGTATTCATTCACCTAATAAAGATTATAGAAGTACAAAATGGAAGGATCTGTTAGAACCTGGTGTA
AAACGTGCATTGGTCGTGGGTGTTGGAATACAAGTACTTCAACAGTTTGCTGGCATCAATGGCATTCTT
TATTACACTCCACAAATACTTGAGCAAGCTGGTGTTGGGGTTCTTCTCTCAAAGTTTGGCATCAACTCT
TCTTCAGTGTCGATTCTTATGAGCGCTCTCACAACTTTATTGATGCTTCCTTTTATTTGCATAGCCATGTG
GCTTATGGACCGCTCTGGAAGAAGAAGGATACTACTTGTGACAATACCCATCTTGGTTGTGTCCCTCAT
TGTTTTGGTAACAGTCAACATTGTGAATCTGAGTGCTGAACTACACGCACTGCTCTCAACAATGAGTG
TTGGAATCTATTTCTGCATATTTGTCATGGGTTTTGGTCCAATACCAAATATATTCTGCTCGGAGATTTTC
CCCAACAAAGTTCGTGCTATTTGCTTGGCCATATGTAGCCTTATCTTTTGGATTTGTGACATCATTGTGA
CATATACCCTCCCTGTATTGTTGAGGTATATAGGTCTTGCGGGTGTTTTTGGAGTTTATGCCATTGTTTGT
GTGCTGGCTTTTGTTTTCGTTTGTTTAAAGGTCCCGGAAACAAAGAATATACCTATTGAGGTCATAGCA
GAGTTCTATGCACTTGGCGGGTCAGGTACCCAAATCATCCAAGAGAGGCAGAAAGAAAATTCAGAGA
AATTATTAGCGTGA
SEQ ID NO: 12 amino acid sequence of TMT3D

MEDLTRPLVRDSEANLHAKLAPSGSGSGLQQACKKLRDLTTVDRAVLVAFVASIGNLLQGWDNASIAGAM
FYIKDEFKLDSMLMIEGCIMAMALFGATIITTLSGMLSDKFGRWAMLLTSAVLSFVSAVLVIFWSQHVYML
LFARLIQGFSIGLAVTLVPLYIVETAPPDMRGKLSTFPQLSGSVGMFLSYCMVFWMSMLPKVSWRIMLGIQ
LIPSLIYSILTIFYLPETPSWLVSQGRVDEAKMVLQKLRRKEDVSGEMASLLEGTQVGDTPSIEEYLISTNES
MLREKVIGNDEIIKLYGLPEDLHCVAYPLKRTNTEESAIGHSVSRGASFYDPVVNIIGSMHGLPEVAHGIFNE
LEQQGPIEGDEENQEETKEHELEHNRDDTYDSEHDYLIQPKPTNINDFVVCRKSGHIGGGWQLAWKMSSG
YHLDGQMEGGMERVYLHEGGVPSSENLLDAPIDGNFIQATALVNKSVFHKSGHNIGIHSPNKDYRSTKWK
DLLEPGVKRALVVGVGIQVLQQFAGINGILYYTPQILEQAGVGVLLSKFGINSSSVSILMSALTTLLMLPFIC
IAMWLMDRSGRRRILLVTIPILVVSLIVLVTVNIVNLSAELHALLSTMSVGIYFCIFVMGFGPIPNIFCSEIFPN
KVRAICLAICSLIFWICDIIVTYTLPVLLRYIGLAGVFGVYAIVCVLAFVFVCLKVPETKNIPIEVIAEFYALG
GSGTQIIQERQKENSEKLLA

## Claims

1. A method for producing a modified wheat plant, comprising:
knocking down and/or knocking out *TaMLO-A1, TaMLO-B1* and/or *TaMLO-D1* genes in a wheat plant; and
increasing expression of TaTMT3 protein in the wheat plant,
thereby obtaining a modified wheat plant which has increased resistance to powdery mildew and comparable or preferably increased yield relative to a corresponding wild-type wheat plant.

2. The method of claim 1, wherein the *TaMLO-A1, TaMLO-B1* and/or *TaMLO-D1* genes in the wheat plant is(are) knocked out or knocked down by introducing antisense RNA, amiRNA, siRNA or shRNA targeting the*TaMLO-A1*, *TaMLO-B1* and/or *TaMLO-D1* genes into the wheat plant.

3. The method of claim 1, wherein the*TaMLO-A1*, *TaMLO-B1* and/or *TaMLO-D1* genes in a wheat plant is(are) knocked out or knocked down by introducing a gene editing system, such as a meganuclease, zinc finger nuclease, transcriptional activator-like effector nuclease (TALEN) or CRISPR gene editing system, resulting in mutation(s) in the*TaMLO-A1*, *TaMLO-B1* and/or *TaMLO-D1* genes.

4. The method of claim 3, further comprising a step of obtaining a wheat plant homozygous for the mutation(s) in the *TaMLO-A1, TaMLO-B1* and/or *TaMLO-D1* genes.

5. The method of any one of claims 1-4, wherein the *TaMLO-A1, TaMLO-B1* and *TaMLO-D1* genes in the wheat plant are knocked out.

6. The method of any one of claims 1-5, wherein the expression of TaTMT3 protein is increased by introducing an expression construct comprising a nucleotide sequence encoding the TaTMT3 protein into the wheat plant, wherein the nucleotide sequence encoding the TaTMT3 protein is operably linked to an expression regulatory element; or the expression of TaTMT3 protein is increased by modifying the expression regulatory sequence of endogenous *TaTMT3* gene of the wheat plant.

7. A method for producing a modified wheat plant, comprising:
providing a first wheat plant in which *TaMLO-A1, TaMLO-B1*, and/or *TaMLO-D1* genes is(are) knocked down and/or knocked out;
providing a second wheat plant in which expression of TaTMT3 protein is increased; and
crossing the first wheat plant with the second wheat plant to obtain a modified wheat plant in which the *TaMLO-A1, TaMLO-B1* and/or *TaMLO-D1* genes is(are) knocked down and/or knocked out, and the expression of TaTMT3 protein is increased,
wherein the modified wheat plant has increased resistance to powdery mildew and comparable or preferably increased yield relative to a corresponding wild-type wheat plant.

8. The method of claim 7, wherein the first wheat plant has increased resistance to powdery mildew relative to a corresponding wild-type wheat plant.

9. The method of claim 7 or 8, wherein the second plant has increased yield relative to a corresponding wild-type wheat plant.

10. The method of any one of claims 1-9, wherein the TaMLO-A1 gene encodes an amino acid sequence shown in SEQ ID NO: 2, the TaMLO-B1 gene encodes an amino acid sequence shown in SEQ ID NO: 4, and the TaMLO-D1 gene encodes an amino acid sequence shown in SEQ ID NO: 6.

11. A method for producing a modified wheat plant, comprising increasing expression of TaTMT3 protein in a wheat plant, thereby obtaining a modified wheat plant which has an increased yield relative to a corresponding wild-type wheat plant.

12. The method of claim 11, wherein the expression of TaTMT3 protein is increased by introducing an expression construct comprising a nucleotide sequence encoding the TaTMT3 protein into the wheat plant, wherein the nucleotide sequence encoding TaTMT3 protein is operably linked to an expression regulatory element; or the expression of TaTMT3 protein is increased by modifying the expression regulatory sequence of endogenous *TaTMT3* gene of the wheat plant.

13. The method of any one of claims 1-12, wherein the TaTMT3 protein is TaTMT3B protein, for example, the TaTMT3 protein comprises an amino acid sequence shown in SEQ ID NO: 8.

14. A modified wheat plant or progeny or parts thereof, wherein the wheat plant can be produced or is produced by the method of any one of claims 1-13.

15. A modified wheat plant or progeny or parts thereof, wherein in the modified wheat plant, *TaMLO-A1, TaMLO-B1* and/or *TaMLO-D1* genes is(are) knocked down and/or knocked out, and expression of TaTMT3 protein is increased, and the modified wheat plant has increased resistance to powdery mildew and comparable or preferably increased yield relative to a wild-type wheat plant.

16. A modified wheat plant or progeny or parts thereof, wherein the modified wheat plant has increased expression of TaTMT3 protein and has increased yield relative to a wild-type wheat plant.

17. The method of any one of claims 1-13 or the modified wheat plant or progeny or parts thereofof any one of claims 14-16, wherein the wheat plant is selected from the group consisting of Triticum aestivum, T. aethiopicum, T. araraticum, T. boeoticum, T. carthlicum, T. compactum, T. dicoccoides, T. dicoccum, T. durum, T. ispahanicum, T. karamyschevii, T. macha, T. militinae, T. monococcum, T. polonicum, T. repens, T. spelta, T. sphaerococcum, T. timopheevii, T. turanicum, T. turgidum, T. urartu, T. vavilovii and T. zhukovskyi, and preferably, the wheat plant is Triticum aestivum, in particular cultivar Bobwhite.
